(19) Europäisches Patentamt
European Patent Office
Office européen des brevets

(11) Veröffentlichungsnummer: **0 622 358 A1**

(12) # EUROPÄISCHE PATENTANMELDUNG

(21) Anmeldenummer: **94103881.2**

(22) Anmeldetag: **14.03.94**

(51) Int. Cl.[5]: **C07D 233/68**, C07D 233/60, C07D 233/90, C07D 401/12, A61K 31/415

(30) Priorität: **26.03.93 DE 4309968**

(43) Veröffentlichungstag der Anmeldung:
**02.11.94 Patentblatt 94/44**

(84) Benannte Vertragsstaaten:
**AT BE CH DE DK ES FR GB GR IE IT LI LU MC NL PT SE**

(71) Anmelder: **BAYER AG**

**D-51368 Leverkusen (DE)**

(72) Erfinder: **Müller, Ulrich, Dr.**
**Neuer Triebel 91**
**D-42111 Wuppertal (DE)**
Erfinder: **Dressel, Jürgen, Dr.**
**Tuchstrasse 48**
**D-42477 Radevormwald (DE)**
Erfinder: **Fey, Peter, Dr.**
**Am Eickhof 23**
**D-42111 Wuppertal (DE)**
Erfinder: **Hanko, Rudolf, Dr.**
**Waldsaum 25**
**D-45134 Essen (DE)**
Erfinder: **Hübsch, Walter, Dr.**
**Wildsteig 22**
**D-42113 Wuppertal (DE)**
Erfinder: **Krämer, Thomas, Dr.**
**Schneewittchenweg 37**
**D-42111 Wuppertal (DE)**
Erfinder: **Müller-Gliemann, Matthias, Dr.**
**Laibacher Strasse 10**
**D-42697 Solingen (DE)**
Erfinder: **Beuck, Martin, Dr.**
**Trills 7**
**D-40699 Erkrath (DE)**
Erfinder: **Kazda, Stanislav, Prof. Dr.**
**Gellertweg 18**
**D-42115 Wuppertal (DE)**
Erfinder: **Wohlfeil, Stefan, Dr.**
**Tucherweg 25**
**D-40724 Hilden (DE)**
Erfinder: **Knorr, Andreas, Dr.**
**Trillser Graben 10**
**D-40699 Erkrath (DE)**
Erfinder: **Stasch, Johannes-Peter, Dr.**
**Alfred-Nobel-Strasse 109**
**D-42651 Solingen (DE)**
Erfinder: **Zaiss, Siegfried, Dr.**
**Farnweg 3**
**D-42113 Wuppertal (DE)**

(54) **Phenylglycinamide von 4-Imidazolylmethyl-Phenylessigsäuren und ihre Verwendung gegen Bluthochdruck und Atherosklerose.**

(57) Phenylglycinamide der allgemeinen Formel

EP 0 622 358 A1

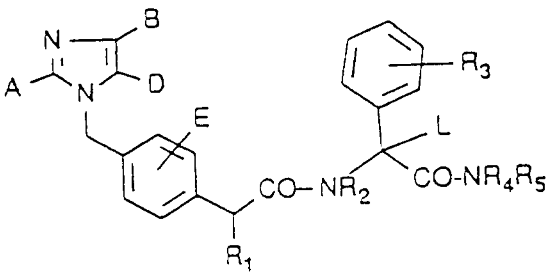

(I),

sowie diese Verbindungen enthaltende Arzneimittel zur Behandlung von arteriellem Bluthochdruck und Atherosklerose.

Die Erfindung betrifft Phenylglycinamide von heterocyclisch substituierten Phenylessigsäurederivaten, Verfahren zu ihrer Herstellung sowie ihre Verwendung in Arzneimitteln, insbesondere als blutdrucksenkende und antiatherosklerotische Mittel.

Es ist bekannt, daß Renin, ein proteolylisches Enzym, in vivo vom Angiotensinogen das Dekapeptid Angiotensin I abspaltet, welches wiederum in der Lunge, den Nieren oder anderen Geweben zu dem blutdrucksteigerndes Oktapeptid Angiotensin II abgebaut wird. Die verschiedenen Effekte des Angiotensin II, wie beispielsweise Vasokonstriktion, $Na^+$-Retention in der Niere, Aldosteronfreisetzung in der Nebenniere und Tonuserhöhung des sympathischen Nervensystems wirken synergistisch im Sinne einer Blutdruckerhöhung.

Darüber hinaus besitzt Angiotensin II die Eigenschaft, das Wachstum und die Vermehrung von Zellen wie beispielsweise von Herzmuskelzellen und glatten Muskelzellen zu fördern, wobei diese bei verschiedenen Krankheitzuständen (z.B. Hypertonie, Atherosklerose und Herzinsuffizienz) vermehrt wachsen und proliferieren.

Ein möglicher Ansatz zum Eingriff in das Renin-Angiotensin-System (RAS) ist neben der Inhibierung der Reninaktivität die Hemmung der Aktivität des Angiotensin-Konversionsenzyms (ACE) sowie die Blockade von Angiotensin II-Rezeptoren.

Die vorliegende Erfindung betrifft neue Phenylglycinamide von heterocyclisch substituierten Phenylessigsäurederivaten der allgemeinen Formel (I)

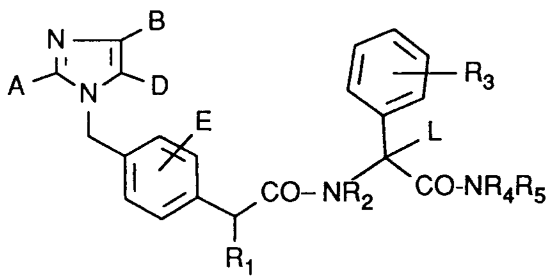

(I),

in welcher

| | |
|---|---|
| A | für geradkettiges oder verzweigtes Alkyl oder Alkenyl mit jeweils bis zu 8 Kohlenstoffatomen steht, oder<br>für Cycloalkyl mit 3 bis 8 Kohienstoffatomen steht, |
| B | für Wasserstoff, Halogen oder für Perfluoralkyl mit bis zu 5 Kohlenstoffatomen steht, |
| D | für eine Gruppe der Formel-$CH_2$-$OR^6$ oder -CO-$R^7$ steht,<br>worin |
| $R^6$ | Wasserstoff oder geradkettiges oder verzweigtes Alkyl mit bis zu 8 Kohlenstoffatomen bedeutet, |
| $R^7$ | Wasserstoff, Hydroxy oder geradkettiges oder verzweigtes Alkoxy mit bis zu 8 Kohlenstoffatomen bedeutet, |
| E | für Wasserstoff, Halogen, Nitro, Hydroxy, Trifluormethyl, Trifluormethoxy, geradkettiges oder verzweigtes Alkyl, Alkoxy oder Alkoxycarbonyl mit jeweils bis zu 6 Kohlenstoffatomen, Cyano oder Carboxy steht, |
| L | für Wasserstoff oder geradkettiges oder verzweigtes Alkyl mit bis zu 4 Kohlenstoffatomen steht, |
| $R^1$ | für Cycloalkyl mit 3 bis 8 Kohlenstoffatomen steht, oder<br>für geradkettiges oder verzweigtes Alkyl mit bis zu 8 Kohlenstoffatomen steht, das gegebenenfalls durch Cycloalkyl mit 3 bis 8 Kohlenstoffatomen substituiert ist, |
| $R^2$ | für Wasserstoff oder für geradkettiges oder verzweigtes Alkyl mit bis zu 6 Kohlenstoffatomen steht, |
| $R^3$ | für Wasserstoff, Hydroxy, Halogen oder für geradkettiges oder verzweigtes Alkoxy mit bis zu 6 Kohlenstoffatomen steht, |
| $R^4$ und $R^5$ | gleich oder verschieden sind und für Wasserstoff, Phenyl, Pyridyl, Cycloalkyl mit 3 bis 8 Kohlenstoffatomen, oder für geradkettiges oder verzweigtes Alkyl mit bis zu 8 Kohlenstoffatomen stehen, das gegebenenfalls durch Hydroxy, Pyridyl, Carboxy, geradkettiges oder verzweigtes Alkoxycarbonyl mit bis zu 6 Kohlenstoffatomen oder durch eine Gruppe der Formel -$NR^8R^9$ substituiert ist,<br>worin |

$R^8$ und $R^9$ gleich oder verschieden sind und Wasserstoff oder geradkettiges oder verzweigtes Alkyl mit bis zu 4 Kohlenstoffatomen bedeuten

oder

$R^4$ und $R^5$ gemeinsam mit dem Stickstoffatom einen 6-gliedrigen gesättigten Heterocyclus mit bis zu 3 Heteroatomen aus der Reihe S, N oder O bilden,

und deren Salze.

Die erfindungsgemäßen Verbindungen der allgemeinen Formel (I) können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der neuen Phenylglycinamide von heterocyclisch substituierten Phenylessigsäurederivaten können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe besitzen. Besonders bevorzugt sind z.B. Natrium-, Kalium-, Magnesium- oder Calciumsalze, sowie Ammoniumsalze, die abgeleitet sind von Ammoniak, oder organischen Aminen wie beispielsweise Ethylamin, Di- bzw. Triethylamin, Di- bzw. Triethanolamin, Dicyclohexylamin, Dimethylaminoethanol, Arginin, Lysin oder Ethylendiamin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweiligen Mischungen. Die Racemformen lassen sich ebenso wie die Diastereomeren in bekannter Weise in die stereoisomer einheitlichen Bestandteile trennen [vgl. E.L. Eliel, Stereochemistry of Carbon Compounds, McGraw Hill, 1962].

Bevorzugt sind Verbindungen der allgemeinen Formel (I),

in welcher

A für geradkettiges oder verzweigtes Alkyl oder Alkenyl mit jeweils bis zu 6 Kohlenstoffatomen steht, oder

für Cyclopropyl, Cyclobutyl, Cyclopentyl, Cyclohexyl oder Cycloheptyl steht,

B für Wasserstoff, Fluor, Chlor, Brom oder für Perfluoralkyl mit bis zu 4 Kohlenstoffatomen steht,

D für eine Gruppe der Formel $-CH_2OR^6$ oder $-CO-R^7$ steht,

worin

$R^6$ Wasserstoff oder geradkettiges oder verzweigtes Alkyl mit bis zu 6 Kohlenstoffatomen bedeutet,

$R^7$ Wasserstoff, Hydroxy oder geradkettiges oder verzweigtes Alkoxy mit bis zu 6 Kohlenstoffatomen bedeutet,

E für Wasserstoff, Fluor, Chlor, Brom, Trifluormethyl, Carboxy oder geradkettiges oder verzweigtes Alkyl, Alkoxy oder Alkoxycarbonyl mit jeweils bis zu 4 Kohlenstoffatomen steht,

L für Wasserstoff oder Methyl steht,

$R^1$ für Cyclopropyl, Cyclopentyl, Cyclohexyl oder Cycloheptyl steht, oder für geradkettiges oder verzweigtes Alkyl mit bis zu 4 Kohlenstoffatomen steht, das gegebenenfalls durch Cyclopropyl, Cyclopentyl, Cylcohexyl oder Cycloheptyl substituiert ist,

$R^2$ für Wasserstoff oder für geradkettiges oder verzweigtes Alkyl mit bis zu 4 Kohlenstoffatomen steht,

$R^3$ für Wasserstoff, Hydroxy, Fluor, Chlor, Brom oder für geradkettiges oder verzweigtes Alkoxy mit bis zu 4 Kohlenstoffatomen steht,

$R^4$ und $R^5$ gleich oder verschieden sind und für Wasserstoff, Pyridyl, Cyclopentyl, Cyclohexyl, Phenyl oder für geradkettiges oder verzweigtes Alkyl mit bis zu 6 Kohlenstoffatomen stehen, das gegebenenfalls durch Hydroxy, Pyridyl, Carboxy, geradkettiges oder verzweigtes Alkoxycarbonyl mit bis zu 4 Kohlenstoffatomen oder durch eine Gruppe der Formel $-NR^8R^9$ substituiert ist,

worin

$R^8$ und $R^9$ gleich oder verschieden sind und Wasserstoff oder Methyl bedeuten,

oder

$R^4$ und $R^5$ gemeinsam mit dem Stickstoffatom einen Morpholin-, Piperazin-oder Piperidinring bilden,

und deren Salze.

Besonders bevorzugt sind Verbindungen der allgemeinen Formel (I),

in welcher

A für geradkettiges oder verzweigtes Alkyl oder Alkenyl mit jeweils bis zu 4 Kohlenstoffatomen steht, oder für Cyclopropyl, Cyclopentyl oder Cyclohexyl steht,

B für Wasserstoff, Fluor, Chlor oder für Perfluoralkyl mit bis zu 2 Kohlenstoffatomen steht,

D für eine Gruppe der Formel $-CH_2OR^6$ oder $-CO-R^7$ steht,

worin

$R^6$ Wasserstoff oder geradkettiges oder verzweigtes Alkyl mit bis zu 4 Kohlenstoffatomen bedeutet,

$R^7$ Wasserstoff, Hydroxy oder geradkettiges oder verzweigtes Alkoxy mit bis zu 4 Kohlenstoffatomen bedeutet,

E für Wasserstoff, Fluor, Chlor, Brom, Trifluormethyl, Trifluormethoxy oder Methyl steht,

L für Wasserstoff oder Methyl steht,

$R^1$ für Cyclopentyl, Cyclohexyl oder Cycloheptyl steht, oder für geradkettiges oder verzweigtes Alkyl mit bis zu 3 Kohlenstoffatomen steht, das gegebenenfalls durch Cyclopentyl, Cyclohexyl oder Cycloheptyl substituiert ist,

$R^2$ für Wasserstoff, Methyl oder Ethyl steht,

$R^3$ für Wasserstoff, Hydroxy, Fluor, Chlor, Brom oder Methoxy steht,

$R^4$ und $R^5$ gleich oder verschieden sind und für Wasserstoff, Pyridyl, Cyclohexyl, Phenyl oder für geradkettiges oder verzweigtes Alkyl mit bis zu 4 Kohlenstoffatomen stehen, das gegebenenfalls durch Hydroxy, Pyridyl, Carboxy, geradkettiges oder verzweigtes Alkoxycarbonyl mit bis zu 3 Kohlenstoffatomen, Amino oder Dimethylamino substituiert ist,

oder

$R^4$ und $R^5$ gemeinsam mit dem Stickstoffatom einen Morpholin-, Piperazin oder Piperidinring bilden,

und deren Salze.

Außerdem wurden Verfahren zur Herstellung der erfindungsgemäßen Verbindungen der allgemeinen Formel (I) gefunden, dadurch gekennzeichnet, daß man

[A] entweder Verbindungen der allgemeinen Formel (II)

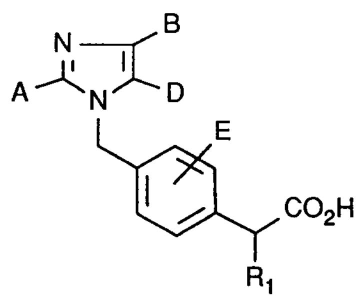

(II),

in welcher

A, B, D, E und $R^1$ die oben angegebene Bedeutung haben,

mit Verbindungen der allgemeinen Formel (III)

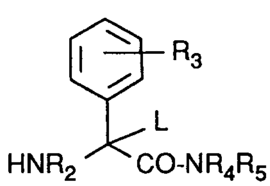

(III),

in welcher

L, $R^2$, $R^3$, $R^4$ und $R^5$ die oben angegebene Bedeutung haben,

oder

[B] Verbindungen der allgemeinen Formel (IV)

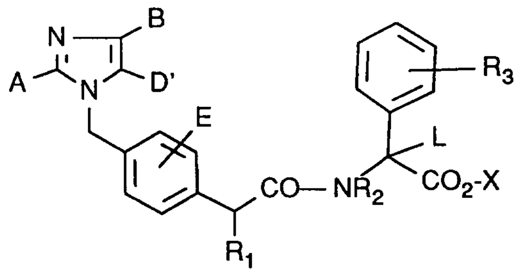

(IV),

in welcher

L, A, B, $R^1$, $R^2$ und $R^3$ die oben angegebene Bedeutung haben,

D' für die $-CH_2-OH$-Gruppe steht

und

X für $C_1$-$C_4$-Alkyl steht,

mit Aminen bzw. Ammoniak der allgemeinen Formel (V)

$HNR^4R^5$ (V),

in welcher

$R^4$ und $R^5$ die oben angegebene Bedeutung haben,

in inerten Lösemitteln, in Anwesenheit einer Base und eines Hilfsstoffes umsetzt,

und im Fall, daß $R^2$, $R^4$ und/oder $R^5$ $\neq$ H eine Alkylierung anschließt,

und die Substituenten A, B, D und E nach üblichen Methoden, wie beispielsweise Reduktion, überführt, und gegebenenfalls die Isomeren trennt, und im Fall der Herstellung der Salze mit einer entsprechenden Base oder Säure umsetzt.

Das erfindungsgemäße Verfahren kann durch folgendes Formelschema beispielhaft erläutert werden:

[A]

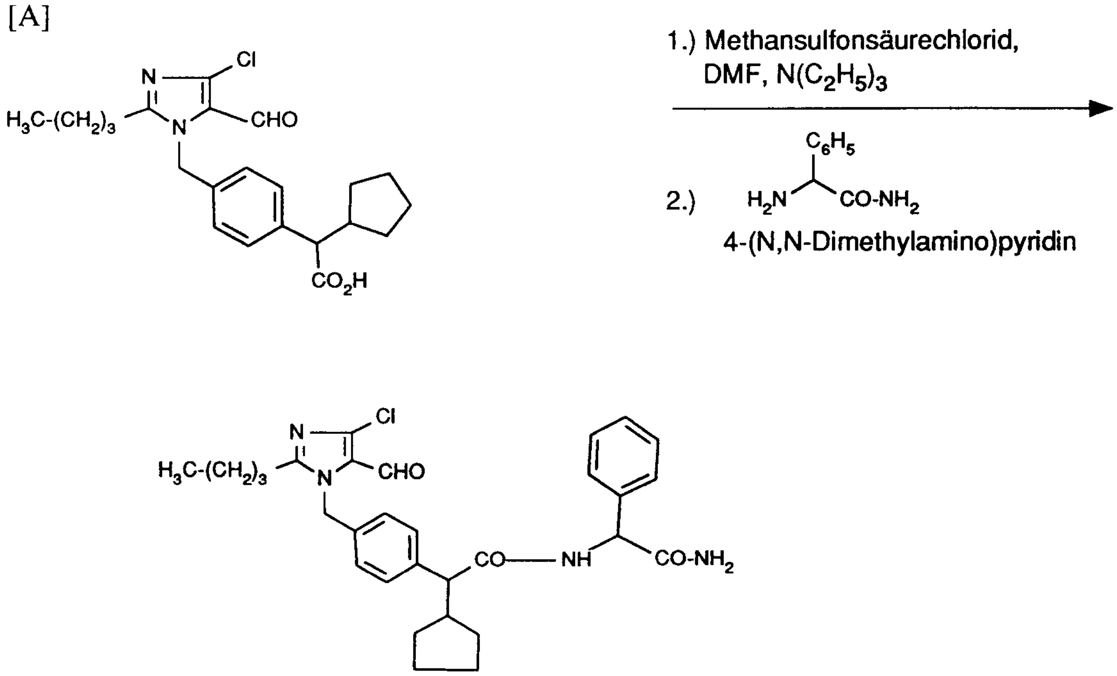


[B]

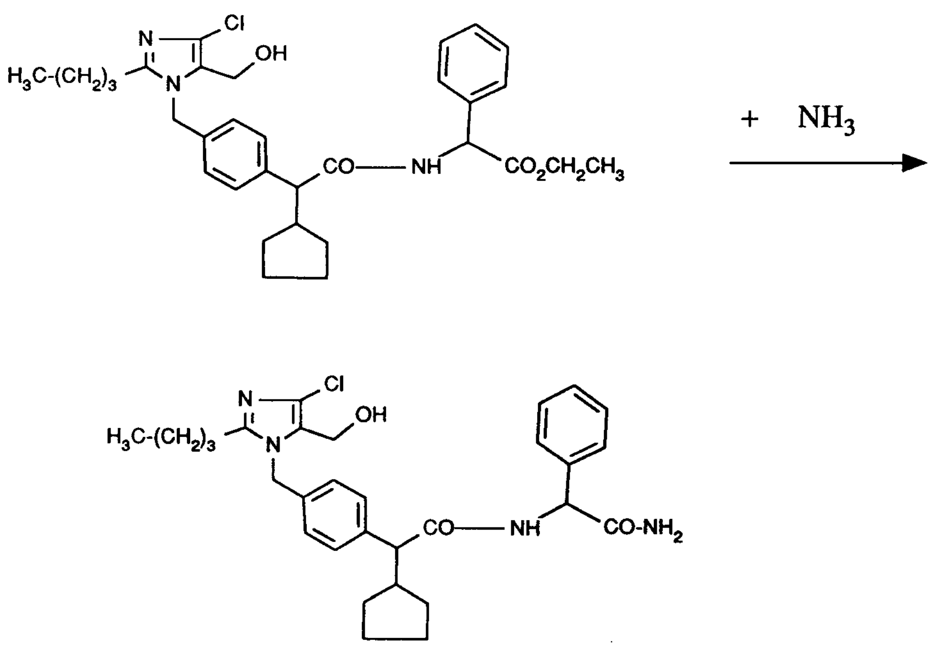


Als Lösemittel für das Verfahren eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt sind Dimethylformamid und Tetrahydrofuran.

Als Basen für das erfindungsgemäße Verfahren können im allgemeinen anorganische oder organische Basen eingesetzt werden. Hierzu gehören vorzugsweise Alkalihydroxide wie zum Beispiel Natriumhydroxid

oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat, oder Alkalialkoholate wie Natrium- oder Kaliummethanolat, Natrium- oder Kallumethanolat oder Kalium-tert.butylat, oder organische Amine (Trialkyl($C_1$-$C_6$)amine) wie Triethylamin, oder Heterocyclen wie 1,4-Diazabicyclo[2.2.2]octan (DABCO), 1,8-Diazabicyclo[5.4.0]undec-7-en (DBU), Pyridin, 4-(N,N-Dimethylamino)pyridin, Methylpiperidin oder Morpholin. Es ist auch möglich, als Basen Alkalimetalle wie Natrium oder deren Hydride wie Natriumhydrid einzusetzen. Bevorzugt sind Natriumhydrid, Kaliumcarbonat, Triethylamin, Pyridin, Kalium-tert.butylat und 4-(N,N-Dimethylamino)pyridin.

Im allgemeinen setzt man die Base in einer Menge von 0,05 mol bis 10 mol, bevorzugt von 1 mol bis 2 mol, bezogen auf 1 mol der Verbindung der Formel (III), ein.

Das erfindungsgemäße Verfahren wird im allgemeinen in einem Temperaturbereich von -30 °C bis +100 °C, bevorzugt von -30 °C bis +60 °C, durchgeführt.

Das erfindungsgemäße Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Die Amidierung erfolgt im allgemeinen in einem der oben aufgeführten Lösemittel, vorzugsweise in N,N-Dimethylformamid.

Die Amidierung erfolgt im allgemeinen gegebenenfalls über die aktivierte Stufe der Säurehalogenide oder gemischter Anhydride, die aus den entsprechenden Säuren durch Umsetzung mit Thionylchlorid, Phosphortrichlorid, Phosphorpentachlorid, Phosphortribromid oder Oxalylchlorid oder Methansulfonsäurechlorid hergestellt werden können.

Die Amidierung oder Acylsulfonamidierung erfolgt im allgemeinen in einem Temperaturbereich von -50 °C bis +80 °C, vorzugsweise von -30 °C bis +20 °C, und Normaldruck.

Als Basen eignen sich dafür neben den oben aufgeführten Basen vorzugsweise Triethylamin und/oder Dimethylaminopyridin, DBU oder DABCO.

Die Base wird in einer Menge von 0,5 mol bis 10 mol, bevorzugt von 1 mol bis 5 mol, bezogen auf 1 mol der entsprechenden Carbonsäure, eingesetzt.

Als säurebindende Mittel für die Amidierung oder Sulfoamidierung können Alkalicarbonate wie Natriumcarbonat, Kaliumcarbonat, Alkali- hydroxide wie beispielsweise Natrium- oder Kaliumhydroxid, oder organische Basen wie Pyridin, Triethylamin, N-Methylpiperidin, oder bicyclische Amidine wie 1,5-Diazabicyclo-[4.3.0]-nonene-5 (DBN) oder 1,5-Diazabicyclo[5.4.0]undecene-5 (DBU) eingesetzt werden. Bevorzugt ist Triethylamin.

Als Dehydratisierungsreagenzien eignen sich Carbodiimide wie beispielsweise Diisopropylcarhodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphonsäureanhydrid oder Isobutylchloroformat oder Benzotriazolyloxy-tris-(dimethylamino)phosphonium-hexafluorophosphat oder Phosphonsäurediphenylesteramid oder Methansulfonsäurechlorid, gegebenenfalls in Anwesenheit von Basen wie Triethylamin oder N-Ethylmorpholin oder N-Methylpiperidin oder Dicyclohexylcarbodiimid und N-Hydroxysuccinimid.

Die säurebindenden Mittel und Dehydratisierungsreagenzien werden im allgemeinen in einer Menge von 0,5 bis 3 mol, bevorzugt von 1 bis 1,5 mol, bezogen auf 1 mol der entsprechenden Carbonsäuren, eingesetzt.

Die Reduktion von Alkoxycarbonylverbindungen oder Aldehyden zu den entsprechenden Alkoholen erfolgt im allgemeinen mit Hydriden, wie Lithiumaluminiumhydrid oder Natriumborhydrid, bevorzugt mit Lithiumaluminiumhydrid in inerten Lösemitteln wie Ethern, Kohlenwasserstoffen oder Alkoholen oder deren Gemischen, bevorzugt in Ethern wie beispielsweise Diethylether, Tetrahydrofuran oder Dioxan, oder Alkoholen wie Ethanol, im Fall der Aldehyde bevorzugt mit Natriumborhydrid in Ethanol, in einem Temperaturbereich von 0 °C bis +150 °C, bevorzugt von +20 °C bis +100 °C, bei Normaldruck.

Die Alkylierung erfolgt im allgemeinen in einem der oben aufgeführten Lösemitteln mit Alkylierungsmitteln wie beispielsweise ($C_1$-$C_8$)-Alkylhalogenide, Sulfonsäureester oder substituierte oder unsubstituierte ($C_1$-$C_6$)-Dialkyl- oder ($C_1$-$C_{10}$)-Diarylsulfate, vorzugsweise Methyljodid, p-Toluolsulfonsäureester oder Dimethylsulfat.

Die Verbindungen der allgemeinen Formel (II) sind neu und können hergestellt werden, indem man Verbindungen der allgemeinen Formel (VI)

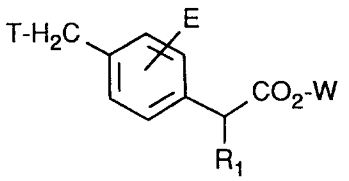

(VI),

in welcher

E und $R^1$ die oben angegebene Bedeutung haben,

T für eine typische Abgangsgruppe wie beispielsweise Chlor, Brom, Jod, Tosylat oder Mesylat, vorzugsweis für Brom steht, und

W für geradkettiges oder verzweigtes ($C_1$-$C_4$)-Alkyl steht,

zunächst mit Imidazolen der allgemeinen Fomel (VII)

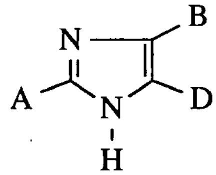

(VII),

in welcher

A, B und D die oben angegebene Bedeutung haben,

in einem der oben aufgeführten inerten Lösemitteln, gegebenenfalls in Anwesenheit einer Base und unter Schutzgasatmosphäre umsetzt,

und in einem letzten Schritt die Ester nach üblichen Methoden verseift.

Als Lösemittel für die Umsetzung mit den Verbindungen der allgemeinen Formel (IV) eignet sich bevorzugt Dimethylformamid.

Als Basen werden bevorzugt Natriumhydrid, Kaliumcarbonat, Triethylamin, Pyridin und Kalium-tert.butylat eingesetzt.

Im allgemeinen setzt man die Base in einer Menge von 0,05 mol bis 10 mol, bevorzugt von 1 mol bis 2 mol, bezogen auf 1 mol der Verbindung der Formel (IV), ein.

Das erfindungsgemäße Verfahren wird im allgemeinen in einem Temperaturbereich von -30 °C bis +100 °C, bevorzugt von -10 °C bis +60 °C, durchgeführt.

Das erfindungsgemäße Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Als Basen eignen sich für die Hydrolyse die üblichen anorganischen Basen. Hierzu gehören bevorzugt Alkalihydroxide oder Erdalkalihydroxide wie beispielsweise Lithiumhydroxid, Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriummethanolat, Natriumethanolat, Kaliummethanolat, Kaliumethanolat oder Kalium-tert.butanolat. Besonders bevorzugt werden Lithiumhydroxid, Natriumhydroxid oder Kaliumthydroxid eingesetzt.

Als Lösemittel eignen sich für die Verseifung Wasser oder die für eine Hydrolyse üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid, oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Bevorzugt erfolgt die Verseifung mit Säuren wie beispielsweise Trifluoressigsäure, Essigsäure, Chlorwasserstoffsäure, Chlorwasserstoffsäure / Dioxan, Bromwasserstoffsäure, Methansulfonsäure, Schwefelsäure oder Perchlorsäure, besonders bevorzugt mit Trifluoressigsäure oder Chlorwasserstoffsäure / Dioxan.

Die Verseigung wird im allgemeinen in einem Temperaturbereich von 0 °C bis +100 °C, bevorzugt von +20 °C bis +80 °C, durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 mol, bevorzugt von 1 bis 1,5 mol, bezogen auf 1 mol des Esters, eingesetzt. Besonders bevorzugt verwendet man molare Mengen der Reaktanden.

Bei der Durchführung der Reaktion entstehen im ersten Schritt die Carboxylate der erfindungsgemäßen Verbindungen als Zwischenprodukte, die isoliert werden können. Die erfindungsgemäßen Säuren erhält man durch Behandeln der Carboxylate mit üblichen anorganischen Säuren. Hierzu gehören bevorzugt Säuren wie beispielsweise Chlorwasserstoffsäure, Bromwasserstoffsäure, Schwefelsäure, Phosphorsäure oder Trifluoressigsäure. Es hat sich bei der Herstellung der Carbonsäuren hierbei als vorteilhaft erwiesen, die basische Reaktionsmischung der Verseifung in einem zweiten Schritt ohne Isolierung der Carboxylate anzusäuern. Die Säuren können dann in üblicher Weise isoliert werden.

Die Verbindungen der allgemeinen Formeln (III), (V) und (VII) sind bekannt.

Die Verbindungen der allgemeinen Formel (VI) sind größtenteils neu und können hergestellt werden, indem man die entsprechenden 4-Methylverbindungen im Sinne einer Substitution, beispielsweise durch Halogenisierung in Anwesenheit einer der oben aufgeführten Basen und/oder Hilfstoffe und eines der Lösemittel umsetzt.

Die Verbindungen der allgemeinen Formel (IV) sind an sich neu und können beispielsweise durch Umsetzung der Verbindungen der allgemeinen Formel (II) mit Verbindungen der allgemeinen Formel (VIII)

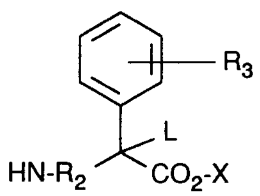 (VIII),

in welcher

L, $R^2$, $R^3$ und X die oben angegebene Bedeutung haben,

wie unter [A] beschrieben, hergestellt werden.

Die Verbindungen der allgemeinen Formel (VIII) sind teilweise bekannt oder können nach üblichen Methoden hergestellt werden.

Die erfindungsgemäßen Verbindungen der allgemeinen Formel (I) zeigen ein nicht vorhersehbares, wertvolles pharmakologisches Wirkspektrum.

Die erfindungsgemäßen Verbindungen besitzen eine spezifische A II-antagonistische Wirkung, da sie kompetitiv die Bindung von Angiotensin II an die Rezeptoren hemmen. Sie unterdrücken die vasokonstriktorischen und Aldosteronsekretionsstimulierenden Effekte des Angiotensin II. Darüber hinaus inhibieren sie die Proliferation von glatten Muskelzellen.

Sie können deshalb in Arzneimitteln zur Behandlung der arteriellen Hypertonie und Atherosklerose eingesetzt werden. Darüber hinaus können sie zur Behandlung von coronaren Herzerkrankungen, Herzinsuffizienz, Störungen der Hirnleistung, ischämischen Gehirnerkrankungen, peripherer Durchblutungsstörungen, Funktionsstörungen der Niere und Nebenniere, bronchospastischen und vaskulär bedingten Erkrankungen der Atemwege, Natriumretention und Ödemen eingesetzt werden.

Untersuchung auf die Hemmung der mit Agonisten induzierten Kontraktion

Kaninchen beiderlei Geschlechts werden durch Nackenschlag betäubt und entblutet, oder fallweise mit Nembutal (ca. 60 - 80 mg/kg i.v.) narkotisiert und durch Öffnung des Thorax getötet. Die Thoraxaorta wird entnommen, von anhaftendem Bindegewebe befreit, in 1,5 mm breite Ringsegmente geteilt und einzeln unter einer Anfangsbelastung von ca. 3,5 g in 10 ml Organbäder mit auf 37 °C temperierter, Carbogen-begaster Krebs-Henseleit-Nährlösung folgender Zusammensetzung: 119 mmol/l NaCl; 2,5 mmol/l $CaCl_2$ x 2 $H_2O$; 1,2 mmol/l $KH_2PO_4$; 10 mmol/l Glucose; 4,8 mmol/l KCl; 1,4 mmol/l $MgSO_4$ x 7 $H_2O$ und 25 mmol/l $NaHCO_3$ verbracht.

Die Kontraktionen werden isometrisch durch Statham UC2-Zellen über Brückenverstärker (ifd Mülheim bzw. DSM Aalen) erfaßt und mittels A/D-Wandler (System 570, Keithley München) digitalisiert sowie ausgewertet. Die Durchführung von Agonistdosiswirkungskurven (DWK) erfolgt stündlich. Mit jeder DWK werden 3 bzw. 4 Einzelkonzentrationen im 4 min-Abstand in die Bäder appliziert. Nach Ende der DWK und darauffolgender Auswaschzyklen (16 mal jeweils ca. 5 sec/min mit der o.a. Nährlösung) schließt sich eine 28-minütige Ruhe- bzw. Inkubationsphase an, innerhalb derer die Kontraktionen in der Regel wieder den

Ausgangswert erreichen.

Die Höhe der im Normalfall 3. DWK wird als Bezugsgröße für die Bewertung der in weiteren Durchgängen zu untersuchenden Testsubstanz verwendet, die bei den nachfolgenden DWK's in jeweils steigender Dosierung mit Beginn der Inktibationszeit in die Bäder appliziert wird. Jeder Aortenring wird dabei ganztägig mit immer dem gleichen Agonisten stimuliert.

Agonisten und ihre Standardkonzentrationen Applikationsvolumen pro Einzelgabe = 100 µl):

| | | |
|---|---|---|
| KCl | 22,7;32,7;42,7;52,7 | mmol/l |
| Noradrenalin | $3x10^{-9}$;$3x10^{-8}$;$3x10^{-7}$;$3x10^{-6}$ | g/ml |
| Serotonin | $10^{-8}$;$10^{-7}$;$10^{-6}$;$10^{-5}$ | g/ml |
| B-HT 920 | $10^{-7}$;$10^{-6}$;$10^{-5}$ | g/ml |
| Methoxamin | $10^{-7}$;$10^{-6}$;$10^{-5}$ | g/ml |
| Angiotensin II | $3x10^{-9}$;$10^{-8}$;$3x10^{-8}$;$10^{-7}$ | g/ml |

Für die Berechnung der $IC_{50}$ (Konzentration bei der die zu untersuchende Substanz eine 50%ige Hemmung verursacht) wird der Effekt jeweils an der 3. = submaximalen Agonistkonzentration zugrundegelegt.

Die erfindungsgemäßen Verbindungen hemmen die durch Angiotensin II induzierte Kontraktion der isolierten Kaninchenaorta dosenabhängig. Die durch Kalium-Depolarisation oder andere Agonisten induzierte Kontraktion wurde nicht oder in hohen Konzentrationen nur schwach gehemmt.

Blutdruckmessungen an der Angiotensin II-infundierten Ratte

Männliche Wistar Ratten (Moellegaard, Kopenhagen, Dänemark) mit einem Körpergewicht von 300 - 350 g, werden mit Thiopental (100 mg/kg i.p.) anästhesiert. Nach Tracheotomie wird in die Femoralarterie ein Katheter zur Blutdruckmessung und in die Femoralvenen ein Katheter für die Angiotensin II-Infusion und ein Katheter für die Substanzverabreichung eingeführt. Nach Gabe des Ganglienblockers Pentolinium (5 mg/kg i.v.) wird die Angiotensin II-Infusion (0,3 µg/kg/min) gestartet Sobald die Blutdruckwerte ein stabiles Plateau erreicht haben, werden die Testsubstanzen entweder intravenös oder als Suspension bzw. Lösung in 0,5% Tylose oral verabreicht. Die Blutdruckveränderungen unter Substanzeinfluß sind als Mittelwerte ± SEM in der Tabelle angegeben.

Bestimmung der antihypertensiven Wirksamkeit bei wachen hypertensiven Ratten

Die orale antihypertensive Wirksamkeit der erfindungsgemäßen Verbindungen wurde an wachen Ratten mit chirurgisch induzierter unilateraler Nierenarterienstenose geprüft. Dazu wurde die rechte Nierenarterie mit einem Silberclip von 0,18 mm lichter Weite eingeengt. Bei dieser Hypertonieform ist die Plasmareninaktivität in den ersten sechs Wochen nach dem Eingriff erhöht.

Der arterielle Blutdruck diese Tiere wurde in definierten Zeitabständen nach Substanzgabe mit der "Schwanzmanschette" unblutig gemessen. Die zu prüfenden Substanzen wurden aufgeschwemmt in einer Tylosesuspension intragastral ("oral") per Schlundsonde in verschiedenen Dosen appliziert. Die erfindungsgemäßen Verbindungen senken den arteriellen Blutdruck der Hochdruckratten in klinisch relevanter Dosierung.

Außerdem hemmen die erfindungsgemäßen Verbindungen die spezifische Bindung von radioaktivem Angiotensin II konzentrationsabhängig.

Interaktion der erfindungsgemäßen Verbindungen mit dem Angiotensin II-Rezeptor an Membranfraktionen der Nebennierenrind (Rind)

Nebennierenrinden vom Rind (NNR), die frisch entnommen und sorgfältig von Mark von Kapsel befreit sind, werden in Sucrose-Lösung (0,32 M) mit Hilfe eines Ultra-Turrax (Janke & Kunkel, Staufen i.B.) zu grobem Membran-Homogenat zerkleinert und in zwei Zentrifugationsschritten zu Membranfraktionen partiell aufgereinigt.

Die Untersuchungen zur Rezeptor-Bindung werden an partiell gereinigten Membranfraktionen boviner NNR mit radioaktivem Angiotensin II in einem Assay-Volumen von 0,25 ml durchgeführt, das im einzelnen die

partiell gereinigten Membranen (50 - 80 µg), $^3H$-Angiotensin II (3-5 nM), Test-Pufferlösung (50 mM Tris, pH 7,2, 5 mM $MgCl_2$ sowie die zu untersuchenden Substanzen enthält. Nach einer Inkubationszeit von 60 min bei Raumtemperatur wird die nicht gebundene Radioaktivität der Proben mittels angefeuchteter Glasfaserfilter (Whatman GF/C) separiert und die gebundene Radioaktivität nach Waschung des Proteins mit eiskalter Pufferlösung (50 mM Tris/HCl, pH 7,4, 5% PEG 6000) in einem Szintillationscocktail spektrophotometrisch gemessen. Die Analyse der Rohdaten erfolgte mit Computer-Programmen zu $K_i$- bzw. $IC_{50}$-Werten ($K_i$: für die verwendete Radioaktivität korrigierte $IC_{50}$-Werte; $IC_{50}$-Werte: Konzentration bei der die zu untersuchende Substanz eine 50%ige Hemmung der spezifischen Bindung des Radioliganden bewirkt).

Untersuchung zur Inhibition der Proliferation glatter Muskelzellen durch die erfindungsgemäßen Verbindungen

Zur Feststellung der antiproliferativen Wirkung der Verbindungen werden glatte Muskelzellen verwendet, die aus den Aorten von Ratten durch die Media-Explantat-Technik gewonnen werden [R. Ross, J. Cell. Biol. 50, 172, 1971]. Die Zellen werden in geeigneten Kulturschalen, in der Regel 96-Loch-Platten, ausgesät und für 2 - 3 Tage in Medium 199 mit 7,5% FCS und 7,5% NCS, 2 mM L-Glutamin und 15 mM HEPES, pH 7,4 in 5% $CO_2$ bei 37 °C kultiviert. Danach werden die Zellen durch Serumentzug für 2 - 3 Tage synchronisiert und sodann mit Serum zum Wachstum angeregt. Gleichzeitig werden Testverbindungen zugesetzt. Nach 16 - 20 Stunden wird 1 µCi $^3H$-Thymidin zugefügt und nach weiteren 4 Stunden der Einbau dieser Substanz in die TCA-präzipitierbare DNA der Zellen bestimmt Zur Bestimmung der $IC_{50}$-Werte wird die Wirkstoffkonzentration errechnet, die bei sequentieller Verdünnung des Wirkstoffes halbmaximale Hemmung der durch 10% FCS hervorgerufene Thymidininkorporation bewirkt.

Tabelle B

| Bsp.-Nr. | $IC_{50}$ [nM] |
|---|---|
| 1 | 1,0 |
| 6 | 0,2 |

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösemittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Laufmittel

(A) Petrolether : Essigester = 3:7
(B) Dichlormethan : Methanol = 8:1
(C) Petrolether : Essigester = 1:4
(D) Petrolether : Essigester = 1:1
(E) Dichlormethan : Methanol = 5:1
(F) Dichlormethan : Essigester = 10:1
(G) Dichlormethan : Methanol = 10:1
(H) Petrolether : Essigester = 2:1
(I) Dichlormethan : Methanol = 20:1

Ausgangsverbindungen

Beispiel I

4-Methylphenylessigsäure-tert.butylester

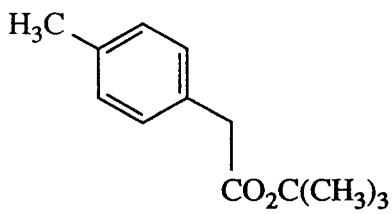

450 g (3 mol) 4-Methylphenylessigsäure, 1,13 l (12 mol) tert.Butanol und 90 g (0,74 mol) Dimethylaminopyridin werden in 2 l Dichlormethan gelöst. Nach Zugabe von 680 g (3,3 mol) Dicyclohexylcarbodiimid, gelöst in 400 ml Dichlormethan, wird 20 h bei 25 °C gerührt, der ausgefallene Harnstoff abgesaugt, mit 200 ml Dichlormethan gewaschen und die organische Phase je zweimal mit 500 ml 2 N Salzsäure und Wasser gewaschen. Die organische Phase wird eingeengt und destilliert.
Ausbeute: 408 g (66% der Theorie)
Siedepunkt: 73 - 78 °C / 0,2 mm

Beispiel II

2-Cyclopentyl-2-(4-methylphenyl)essigsäure-tert.butylester

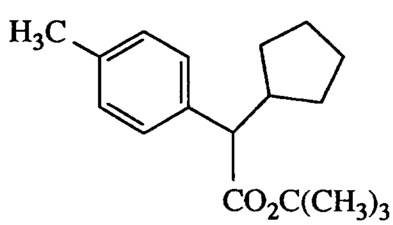

33,5 g (0,3 mol) Kalium-tert.butylat werden in 100 ml DMF unter Feuchtigkeitsausschluß bei 0 °C vorgelegt, und 51,6 g (0,25 mol) 4-Methylphenylessigsäure-tert.butylester in 250 ml DMF zugetropft. Es wird 30 min bei 0 °C gerührt und 32,2 ml (0,3 mol) Cyclopentylbromid in 150 ml DMF bei 5-15 °C zugetropft und 20 h bei 25 °C gerührt. Nach Einengen wird der Rückstand zwischen Wasser/Diethylether verteilt, die Etherphase über Natriumsulfat getrocknet und eingeengt. Das Produkt kristallisiert aus.
Ausbeute: 67 g (97,5% der Theorie)
Festpunkt: 51 - 53 °C

Beispiel III

2-(4-Brommethyl-phenyl)-2-cyclopentyl-essigsäure-tert.butylester

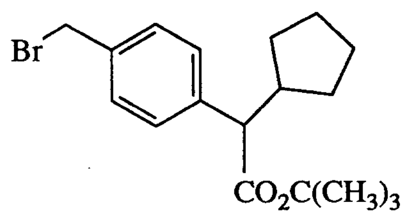

27,4 g (0,1 mol) 2-Cyclopentyl-2-(4-methylphenyl)-essigsäure-tert.butylester werden in 200 ml Tetrachlorkohlenstoff gelöst und zum Sieden erhitzt. Nach Zugabe von 0,82 g Azobisisobutyronitril werden 18,7 g (0,105 mol) N-Bromsuccinimid portionsweise zugegeben und anschließend 1 h refluxiert, auf 0 °C abgekühlt und vom Succinimid abfiltriert. Nach Einengen des Filtrates fallt das Produkt aus. Es wird mit Petrolether (40/60) gewaschen und getrocknet.
Ausbeute: 20 g (57% der Theorie)
Festpunkt: 73 - 76 °C

Beispiel IV

2-[4-(2-Butyl-4-chlor-5-formyl-imidazol-1-yl-methyl)phenyl]-2-cyclopentyl-essigsäure-tert.butylester

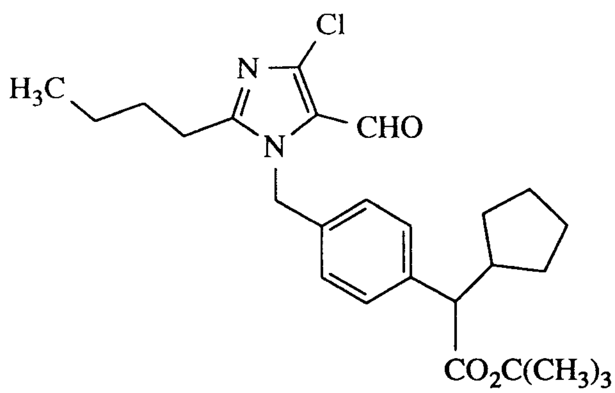

Unter Schutzgas werden 1,6 g (0,053 mol) Natriumhydrid (80%ig) in 50 ml DMF suspendiert, 10 g (0,053 mol) 2-Butyl-5-formyl-4-chlorimidazol (Herstellung nach EP 324 377) in 100 ml DMF bei 0 °C zugetropft, anschließend bei 0 °C 15 min gerührt und 18,9 g (0,053 mol) 2-(4-Brommethylphenyl)-2-cyclopentylessigsäure-tert.-butylester in 100 ml DMF zugetropft. Es wird 2 h bei 0 °C nachgerührt, das Lösemittel abgedampft, der Rückstand in Diethylether aufgenommen, abfiltriert und nach Einengen über Kieselgel 60 mit Dichlormethan chromatographiert.
Ausbeute: 16,2 g (66,7% der Theorie)
Festpunkt: 101-102 °C

Beispiel V

2-[4-(2-Butyl-4-chlor-5-formyl-imidazol-1-yl-methyl)phenyl]-2-cyclopentyl-essigsäure

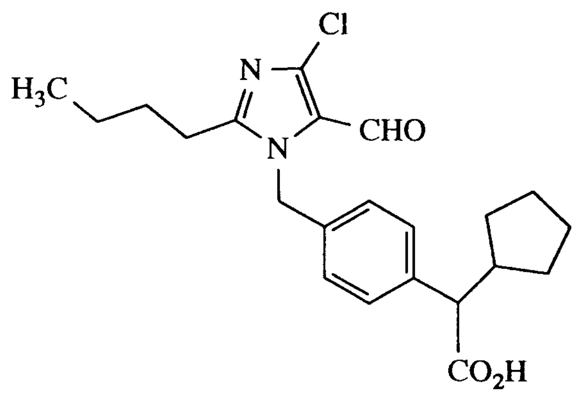

2,3 g (5 mmol) der Verbindung aus Beispiel IV werden in 5 ml Dichiormethan und 5 ml Trifluoressigsäure 5 h bei 25°C gerührt. Nach Einengen wird das Rohprodukt über Kieselgel 60 mit Dichiormethan/Methanol (100:5) chromatographiert.
Ausbeute: 1,8 g (87,6% der Theorie)
Festpunkt: 95-98°C

Beispiel VI

2-{2-[4-(2-Butyl-4-chlor-5-formyl-imidazol-1-yl-methyl)phenyl]-2-cyclopentyl}-acetamido-2-(2-hydroxyphenyl)essigsäuremethylester

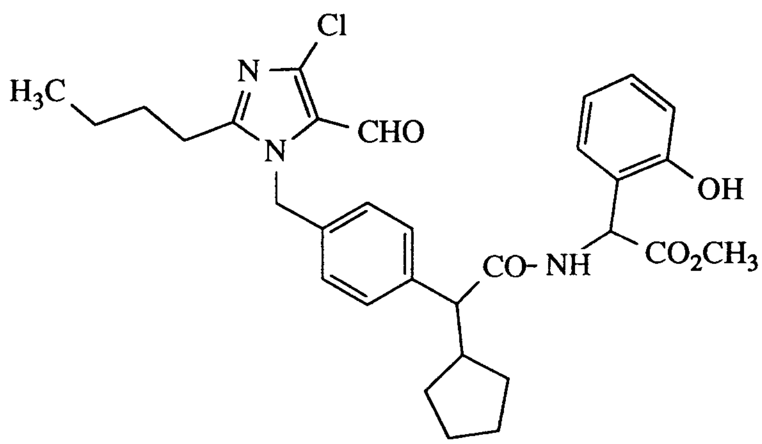

6,0 g (15 mmol) der Verbindung aus Beispiel V werden in 180 ml wasserfreiem Tetrahydrofuran gelöst und bei 0°C mit 4,2 ml (30 mmol) Triethylamin und 1,26 ml (16,5 mmol) Methansulfonylchlorid umgesetzt. Nach 1 Stunde gibt man eine Lösung von 3,92 g (18 mmol) 2-Hydroxyphenylglycinmethylester-Hydrochlorid, 1,82 g (15 mmol) 4-(N,N-Dimethylamino)pyridin und 2,52 ml (18 mmol) Triethylamin in 60 ml Tetrahydrofuran zu und rührt 18 Stunden nach, wobei die Reaktionstemperatur auf Raumtemperatur ansteigt. Das Rohgemisch wird in Wasser gegeben, mit 2 M Salzsäure auf pH = 2 bis 3 gestellt und mit Ether extrahiert. Die organische Phase wird mit Magnesiumsulfat getrocknet und eingedampft. Nach chromatographischer Aufarbeitung an Kieselgel 60 (Merck, Petrolether : Essigester = 2:1) erhält man 4,03 g (7,1 mmol) der Titelverbindung.
$R_f$ = 0,18 (Dichlormethan : Methanol = 50:1)

Herstellungsbeispiele

Beispiel 1

2-{2-[4-(2-Butyl-4-chlor-5-formyl-imidazol-1-yl-methyl)phenyl]-2-cyclopentyl}-acetamido-2-phenyl-acetamid

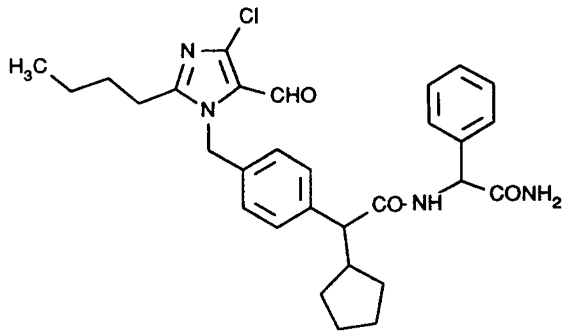

1,54 g (3,8 mmol) der Verbindung aus Beispiel V werden in wasserfreiem N,N-Dimethylformamid bei -30 °C mit 1,06 ml (7,6 mmol) Triethylamin und 0,48 g (4,2 mmol) Methansulfonsäurechlorid umgesetzt. Nach 1 Stunde werden 0,69 g (4,6 mmol) Phenylglycinamid und 0,47 g (3,8 mmol) 4-(N,N-Dimethylamino)pyridin zugegeben und unter langsamer Erwärmung auf Raumtemperatur 24 Stunden nachgerührt. Darauf gießt man in Ether, versetzt mit Wasser und stellt mit 1 M Salzsäure auf pH = 2. Die wäßrige Phase wird mit Ether nachextrahiert und die vereinigten organischen Phasen mehrfach mit 0,01 M Salzsäure gewaschen. Anschließend extrahiert man mehrfach mit wäßriger Natronlauge von pH = 10, wäscht mit Wasser nach, trocknet die organische Phase mit Natriumsulfat und dampft das Lösemittel ab. Das Rohprodukt wird chromatographisch gereinigt (Kieselgel 60, Merck 40-63 µm, Petrolether : Essigester = 1:1).
Ausbeute: 1,56 g (2,9 mmol) Produkt.
$R_f$ = 0,16 und 0,12 (Petrolether : Essigester = 1:1)
In Analogie zur Vorschrift des Beispiels 1 werden die in Tabelle 1 aufgeführten Verbindungen hergestellt:

Tabelle 1:

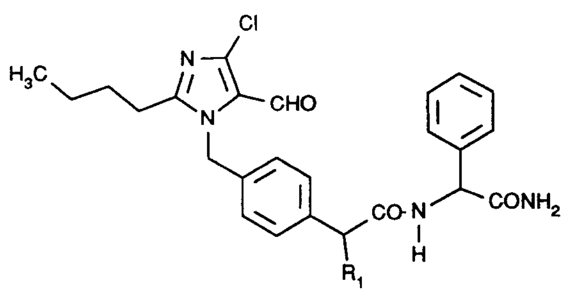

| Bsp.-Nr. | Diastereomer | $R^1$ | $R_f$ (Solvens) |
|---|---|---|---|
| 2 | 4 dia | 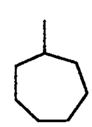 | 0,47 (A) |
| 3 | 4 dia | 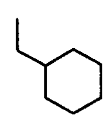 | 0,57 (B) |

Beispiel 4

2-[4-(2-Butyl-4-chlor-5-formyl-imidazol-1-yl-methyl)phenyl]-2-cyclohexyl-essigsäure-phenylglycinamidoamid

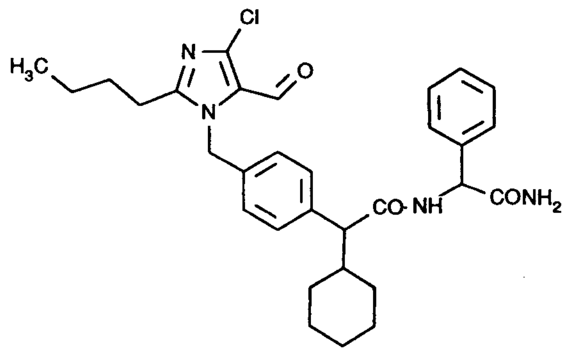

0,42 g (1 mmol) 2-[4-(2-Butyl-4-chlor-5-formyl-imidazol-1-yl-methyl)phenyl]-2-cyclohexylessigsäure werden in 25 ml Dichlormethan bei Raumtemperatur mit 0,2 g (2 mmol) Triethylamin und 0,23 g (1,5 mmol) 1-Hydroxy-1H-benzotriazol in 5 ml Tetrahydrofuran versetzt und auf 0°C gekühlt. Nach Zugabe von 0,31 g (1,5 mmol) Dicyclohexylcarbodiimid in 10 ml Dichlormethan und 30 Minuten Rühren wird eine Lösung von 0,32 g (1,2 mmol) Phenylglycinamid und 0,1 g (1,2 mmol) Triethylamin in 10 ml Dichlormethan zugesetzt und über Nacht bei Raumtemperatur gerührt. Zur Aufarbeitung wird nach Zugabe von Dichlormethan extrahiert, und die vereinigten organischen Phasen werden über Natriumsulfat getrocknet, filtriert, eingeengt und über Kieselgel 60 (Essigester/Petrolether = 1:1) chromatographiert.
Ausbeute: 0,31 g (0,56 mmol) 56% der Theorie
$R_f$ = 0,52 (Dichlormethan / Methanol = 9:1)

Beispiel 5

2-[4-(2-Butyl-4-chlor-5-hydroxymethyl-imidazol-1-yl-methyl)phenyl]-2-cyclohexylessigsäurephenylglycinamido-amid

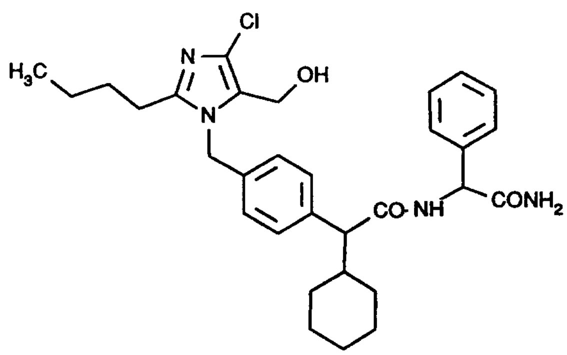

0,15 g (0,3 mmol) 2-[4-(2-Butyl-4-chlor-5-formyl-imidazol-1-yl-methyl)phenyl]-2-cyclohexylessigsäurephenylglycinamido-amid werden in 5 ml Ethanol bei Raumtemperatur mit 10 mg Natriumborhydrid versetzt und zwei Stunden bei Raumtemperatur gerührt. Zur Aufarbeitung wird Wasser zugegeben und mit 1 N Essigsäure auf pH 4-5 eingestellt und dreimal mit Essigester extrahiert. Die vereinigten organischen Phasen werden über Natriumsulfat getrocknet, filtriert und eingeengt.
Ausbeute: 0,16 g (0,29 mmol) 98% der Theorie
$R_f$ = 0,48 (Dichlormethan / Methanol = 9:1)

Beispiel 6

2-{2-[4-(2-Butyl-4-chlor-5-hydroxymethyl-imidazol-1-yl-methyl)phenyl]-2-cyclopentyl}acetamido-2-phenyl-acetamid

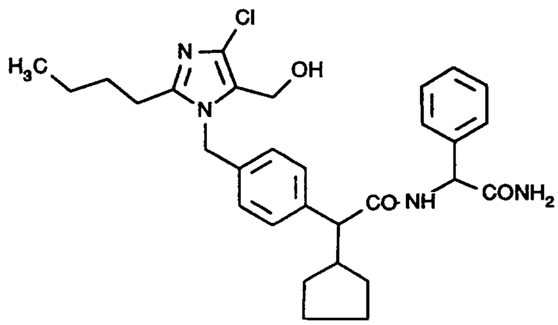

1,37 g (2,6 mmol) der Verbindung aus Beispiel 1 werden bei 20 °C in 15 ml Ethanol mit 0,10 g (2,6 mmol) Natriumboranat umgesetzt. Bei unvollständiger Umsetzung (DC-Kontrolle) wird nach 2 Stunden weiteres Natriumboranat zugegeben. Nach insgesamt 3 Stunden setzt man Wasser und Ether zu, vernichtet überschüssiges Boranat mit Salzsäure bei pH = 2, stellt dann mit 1 M wäßriger Natronlauge pH = 7,5 ein und extrahiert mehrfach mit Ether nach. Die organische Phase wird mit Natriumsulfat getrocknet und eingedampft.
Ausbeute: 1,06 g (2,0 mmol)
$R_f$ = 0,78 und 0,75 (Dichlormethan : Methanol = 5:1)
In Analogie zur Vorschrift des Beispiels 6 werden die in Tabelle 2 aufgeführten Verbindungen hergestellt:

Tabelle 2:

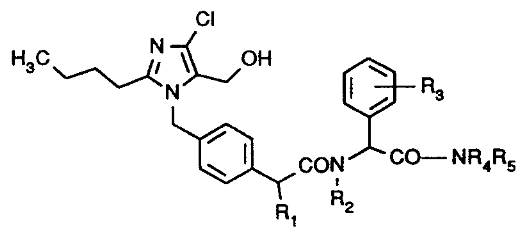

| Bsp.-Nr. | Diastereomer | $R^1$ | $R^2$ | $R^3$ | $R^4$ | $R^5$ | $R_f$ (Solvens) |
|---|---|---|---|---|---|---|---|
| 7 | rac dia A | 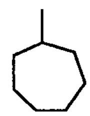 | H | H | H | H | 0,37 (A) |
| 8 | rac dia B | 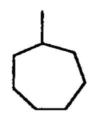 | H | H | H | H | 0,26 (A) |
| 9 | 4 dia | 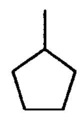 | H | 2-OH | H | H | 0,42 (C) |
| 10 | 4 dia | 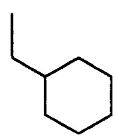 | H | H | H | H | 0,23/0,18 (A) |

Beispiel 11

2-{2-[4-(2-Butyl-4-chlor-5-formyl-imidazol-1-yl-methyl)phenyl]-2-cyclopentyl}-acetamido-2-(2-hydroxyphenyl)-acetamid

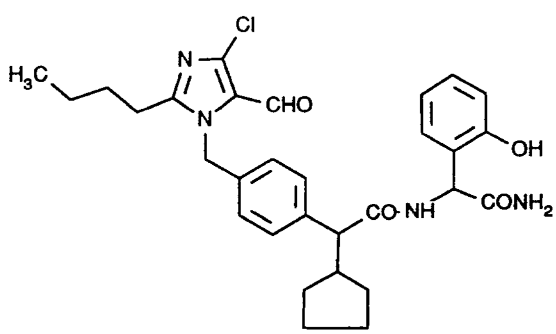

60 mg (0,11 mmol) der Verbindung aus Beispiel VI werden in 2 ml wasserfreiem Tetrahydrofüran gelöst und mit 1 ml 25%iger wäßriger Ammoniaklösung umgesetzt (6 Stunden bei 50 °C). Darauf stellt man mit 0,1 M Schwefelsäure auf pH = 7 und extrahiert mehrfach mit Ether. Die organische Phase wird mit Magnesiumsulfat getrocknet und eingedampft. Nach Entfernung des Restlösemittels im Hochvakuum fallen 40 mg (0,07 mmol) Produkt an.

$R_f$ = 0,21 (Petrolether : Essigester = 1:1)

Die in den Tabellen 3 und 4 aufgeführten Verbindungen sind die getrennten Diastereomere der Beispiele 1 und 2 und können durch Trennung dieser nach üblichen Methoden hergestellt werden:

Tabelle 3:

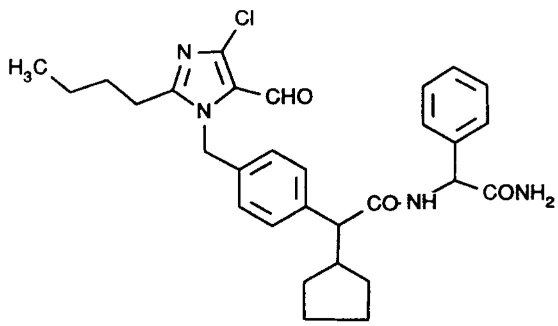

| Bsp.-Nr. | Diastereomer | $R_f$ (Solvens) |
|---|---|---|
| 12 | dia A | 0,16 (D) |
| 13 | dia B | 0,12 (D) |
| 14 | dia C | 0,16 (D) |
| 15 | dia D | 0,12 (D) |

Tabelle 4:

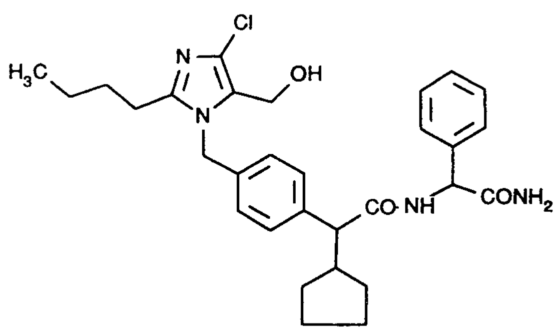

| Bsp.-Nr. | Diastereomer | $R_f$ (Solvens) |
|---|---|---|
| 16 | dia A | 0,78 (E) |
| 17 | dia B | 0,75 (E) |
| 18 | dia C | 0,78 (E) |
| 19 | dia D | 0,75 (E) |

In Analogie zur Vorschrift des Beispiels 11 werden die in Tabelle 5 aufgeführten Verbindungen hergestellt:

Tabelle 5:

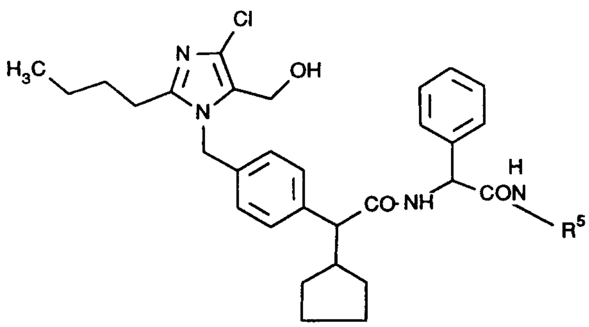

| Bsp.-Nr. | $R^5$ | $R_f$ (LM) |
|---|---|---|
| 20 | $CH_2CO_2CH_2CH_3$ | 0,35 (F) |
| 21 | $CH_2CH_2N(CH_3)_2$ | 0,47 (E) |
| 22 | 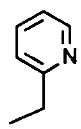 | 0,53 (G) |
| 23 | 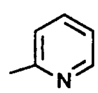 | 0,75 (E) |
| 24 | 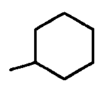 | 0,61 (G) |
| 25 | $CH_2CH_2OH$ | 0,33 (G) |
| 26 | 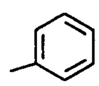 | 0,37 (H) |
| 27 | $CH_3$ | 0,14 (I) |

In Analogie zur Vorschrift des Beispiels 6 werden die in Tabelle 6 aufgeführten Verbindungen hergestellt:

Tabelle 6:

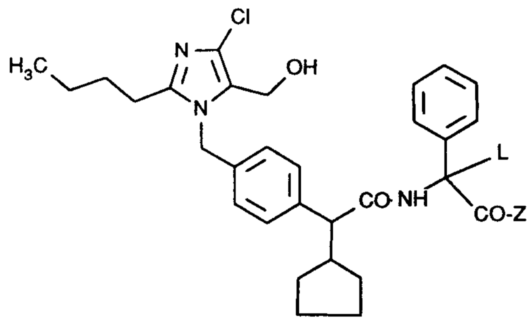

| Bsp.-Nr. | L | Z | $R_f$ (LM) |
|---|---|---|---|
| 28 | $CH_3$ | $NH_2$ | 0,32 (I) |

In Analogie zur Vorschrift des Beispiels 1 werden die in Tabelle 7 aufgeführten Verbindungen hergestellt:

Tabelle 7:

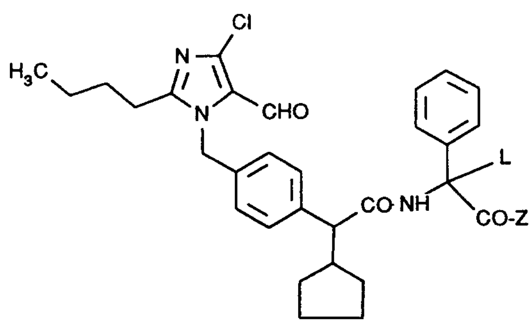

| Bsp.-Nr. | L | Z | $R_f$ (LM) |
|---|---|---|---|
| 29 | $CH_3$ | $NH_2$ | 0,06 (D) |

Beispiel 30

⟨2-{2-[4-(2-Butyl-4-chlor-5-hydroxymethyl-imidazol-1-yl-methyl)phenyl]-2-cyclopentyl}acetamido-2-phenyl-acetamido⟩-essigsäure

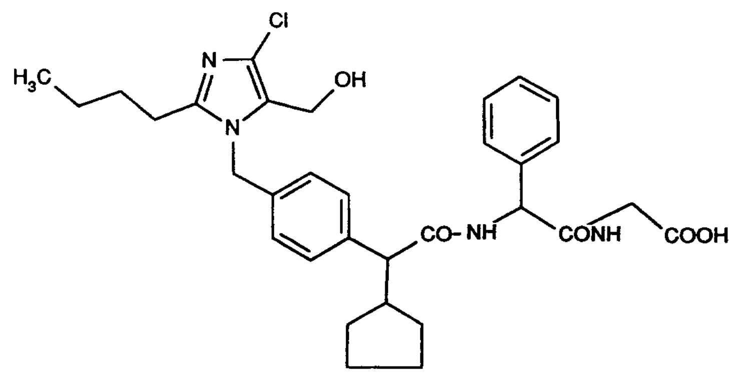

60 mg (0,1 mmol) der Verbindung aus Beispiel 20 werden in 2 ml Ethanol gelöst und bei 22°C 1 Stunde mit 4 ml wäßriger 1M Natronlauge umgesetzt. Das Reaktionsgemisch wird mit Wasser verdünnt, der Ethanolanteil abgedampft und das Produkt durch Ansäuern mit 2M Salzsäure gefällt. Der Niederschlag wird mit Wasser gewaschen und im Hochvakuum über Sicapent (Merck) getrocknet: 43 mg.
$R_f$ = 0,60 (Dichlormethan : Methanol = 10:1)

**Patentansprüche**

**1.** Phenylglycinamide von heterocyclisch substituierten Phenylessigsäurederivaten der allgemeinen Formel

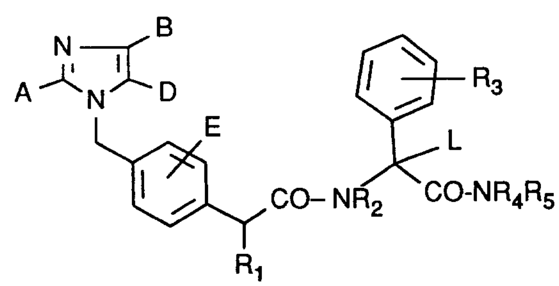

(I),

in welcher

| | |
|---|---|
| A | für geradkettiges oder verzweigtes Alkyl oder Alkenyl mit jeweils bis zu 8 Kohlenstoffatomen steht, oder |
| | für Cycloalkyl mit 3 bis 8 Kohlenstoffatomen steht, |
| B | für Wasserstoff, Halogen oder für Perfluoralkyl mit bis zu 5 Kohlenstoffatomen steht, |
| D | für eine Gruppe der Formel-$CH_2$-$OR^6$ oder -CO-$R^7$ steht, |
| | worin |
| $R^6$ | Wasserstoff oder geradkettiges oder verzweigtes Alkyl mit bis zu 8 Kohlenstoffatomen bedeutet, |
| $R^7$ | Wasserstoff, Hydroxy oder geradkettiges oder verzweigtes Alkoxy mit bis zu 8 Kohlenstoffatomen bedeutet, |
| E | für Wasserstoff, Halogen, Nitro, Hydroxy, Trifluormethyl, Trifluormethoxy, geradkettiges oder verzweigtes Alkyl, Alkoxy oder Alkoxycarbonyl mit jeweils bis zu 6 Kohlenstoffatomen, Cyano oder Carboxy steht, |
| L | für Wasserstoff oder geradkettiges oder verzeigtes Alkyl mit bis zu 4 Kohlenstoffatom steht, |
| $R^1$ | für Cycloalkyl mit 3 bis 8 Kohlenstoffatomen steht, oder |

für geradkettiges oder verzweigtes Alkyl mit bis zu 8 Kohlenstoffatomen steht, das gegebenenfalls durch Cycloalkyl mit 3 bis 8 Kohlenstoffatomen substituiert ist,

$R^2$ für Wasserstoff oder für geradkettiges oder verzweigtes Alkyl mit bis zu 6 Kohlenstoffatomen steht,

$R^3$ für Wasserstoff, Hydroxy, Halogen oder für geradkettiges oder verzweigtes Alkoxy mit bis zu 6 Kohlenstoffatomen steht,

$R^4$ und $R^5$ gleich oder verschieden sind und für Wasserstoff, Pyridyl, Cycloalkyl mit 3 bis 8 Kohlenstoffatomen, Phenyl oder für geradkettiges oder verzweigtes Alkyl mit bis zu 8 Kohlenstoffatomen stehen, das gegebenenfalls durch Hydroxy, Pyridyl, Carboxy, geradkettiges oder verzweigtes Alkoxycarbonyl mit bis zu 6 Kohlenstoffatomen oder durch eine Gruppe der Formel - $NR^8R^9$ substituiert ist,
worin

$R^8$ und $R^9$ gleich oder verschieden sind und Wasserstoff oder geradkettiges oder verzweigtes Alkyl mit bis zu 4 Kohlenstoffatomen bedeuten
oder

$R^4$ und $R^5$ gemeinsam mit dem Stickstoffatom einen 6-gliedrien gesättigen Heterocyclus mit bis zu 3 Heteroatomen aus der Reihe S, N oder O bilden,

und deren Salze.

**2.** Phenylglycinamide von heterocyclisch substituierten Phenylessigsäurederivaten nach Anspruch 1, wobei

A für geradkettiges oder verzweigtes Alkyl oder Alkenyl mit jeweils bis zu 6 Kohlenstoffatomen steht, oder
für Cyclopropyl, Cyclobutyl, Cyclopentyl, Cyclohexyl oder Cycloheptyl steht,

B für Wasserstoff, Fluor, Chlor, Brom oder für Perfluoralkyl mit bis zu 4 Kohlenstoffatomen steht,

D für eine Gruppe der Formel $-CH_2OR^6$ oder $-CO-R^7$ steht,
worin

$R^6$ Wasserstoff oder geradkettiges oder verzweigtes Alkyl mit bis zu 6 Kohlenstoffatomen bedeutet,

$R^7$ Wasserstoff, Hydroxy oder geradkettiges oder verzweigtes Alkoxy mit bis zu 6 Kohlenstoffatomen bedeutet,

E für Wasserstoff, Fluor, Chlor, Brom, Trifluormethyl, Carboxy oder geradkettiges oder verzweigtes Alkyl, Alkoxy oder Alkoxycarbonyl mit jeweils bis zu 4 Kohlenstoffatomen steht,

L für Wasserstoff oder Methyl steht,

$R^1$ für Cyclopropyl, Cyclopentyl, Cyclohexyl oder Cycloheptyl steht, oder
für geradkettiges oder verzweigtes Alkyl mit bis zu 4 Kohlenstoffatomen steht, das gegebenenfalls durch Cyclopropyl, Cyclopentyl, Cylcohexyl oder Cycloheptyl substituiert ist,

$R^2$ für Wasserstoff oder für geradkettiges oder verzweigtes Alkyl mit bis zu 4 Kohlenstoffatomen steht,

$R^3$ für Wasserstoff, Hydroxy, Fluor, Chlor, Brom oder für geradkettiges oder verzweigtes Alkoxy mit bis zu 4 Kohlenstoffatomen steht,

$R^4$ und $R^5$ gleich oder verschieden sind und für Wasserstoff, Pyridyl, Cyclopentyl, Cyclohexyl, Phenyl oder für geradkettiges oder verzweigtes Alkyl mit bis zu 6 Kohlenstoffatomen stehen, das gegebenenfalls durch Hydroxy, Pyridyl, Carboxy, geradkettiges oder verzweigtes Alkoxycarbonyl mit bis zu 4 Kohlenstoffatomen oder durch eine Gruppe der Formel $-NR^8R^9$ substituiert ist,
worin

$R^8$ und $R^9$ gleich oder verschieden sind und Wasserstoff oder Methyl bedeuten
oder

$R^4$ und $R^5$ gemeinsam mit dem Stickstoffatom einen Morpholin-, Piperazin- oder Piperidinring bilden,

und deren Salze.

**3.** Phenylglycinamide von heterocyclisch substituierten Phenylessigsäurederivaten nach Anspruch 1, wobei

| | |
|---|---|
| A | für geradkettiges oder verzweigtes Alkyl oder Alkenyl mit jeweils bis zu 4 Kohlenstoffatomen steht, oder für Cyclopropyl, Cyclopentyl oder Cyclohexyl steht, |
| B | für Wasserstoff, Fluor, Chlor oder für Perfluoralkyl mit bis zu 2 Kohlenstoffatomen steht, |
| D | für eine Gruppe der Formel $-CH_2OR^6$ oder $-CO-R^7$ steht, worin |
| $R^6$ | Wasserstoff oder geradkettiges oder verzweigtes Alkyl mit bis zu 4 Kohlenstoffatomen bedeutet, |
| $R^7$ | Wasserstoff, Hydroxy oder geradkettiges oder verzweigtes Alkoxy mit bis zu 4 Kohlenstoffatomen bedeutet, |
| E | für Wasserstoff, Fluor, Chlor, Brom, Trifluormethyl, Trifluormethoxy oder Methyl steht, |
| L | für Wasserstoff oder Methyl steht, |
| $R^1$ | für Cyclopentyl, Cyclohexyl oder Cycloheptyl steht, oder für geradkettiges oder verzweigtes Alkyl mit bis zu 3 Kohlenstoffatomen steht, das gegebenenfalls durch Cyclopentyl, Cyclohexyl oder Cycloheptyl substituiert ist, |
| $R^2$ | für Wasserstoff, Methyl oder Ethyl steht, |
| $R^3$ | für Wasserstoff, Hydroxy, Fluor, Chlor, Brom oder Methoxy steht, |
| $R^4$ und $R^5$ | gleich oder verschieden sind und für Wasserstoff, Pyridyl, Cyclohexyl, Phenyl oder für geradkettiges oder verzweigtes Alkyl mit bis zu 4 Kohlenstoffatomen stehen, das gegebenenfalls durch Hydroxy, Pyridyl, Carboxy, geradkettiges oder verzweigtes Alkoxycarbonyl mit bis zu 3 Kohlenstoffatomen, Amino oder Dimethylamino substituiert ist, oder |
| $R^4$ und $R^5$ | gemeinsam mit dem Stickstoffatom einen Morpholin-, Piperazin- oder Piperidinring bilden, |

und deren Salze.

4. Phenylglycinamide von heterocyclisch substituierten Phenylessigsäurederivaten als Arzneimittel.

5. Verfahren zur Herstellung von Phenylglycinamiden von heterocyclisch substituierten Phenylessigsäurederivaten nach Anspruch 1 bis 3, dadurch gekennzeichnet, daß man

[A] entweder Verbindungen der allgemeinen Formel (II)

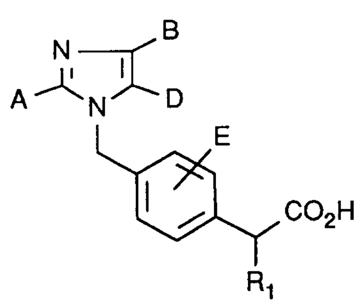

(II),

in welcher

A, B, D, E und $R^1$ die oben angegebene Bedeutung haben,

mit Verbindungen der allgemeinen Formel (III)

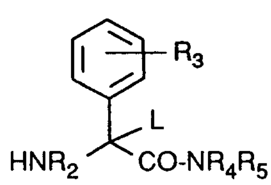

(III),

in welcher

L, $R^2$, $R^3$, $R^4$ und $R^5$ die oben angegebene Bedeutung haben,
oder
[B] Verbindungen der allgemeinen Formel (IV)

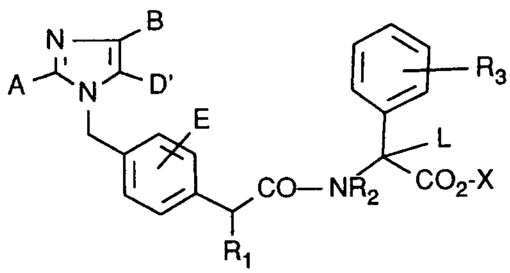

(IV),

in welcher
L, A, B, $R^1$, $R^2$ und $R^3$ die oben angegebene Bedeutung haben,
D' für die $-CH_2-OH$-Gruppe steht
und
X für $C_1$-$C_4$-Alkyl steht,
mit Aminen bzw. Ammoniak der allgemeinen Formel (V)

$HNR^4R^5$ (V),

in welcher
$R^4$ und $R^5$ die oben angegebene Bedeutung haben,
in inerten Lösemitteln, in Anwesenheit einer Base und eines Hilfsstoffes umsetzt,
und im Fall, daß $R^2$, $R^4$ und/oder $R^5 \neq H$ eine Alkylierung anschließt,
und die Substituenten A, B, D und E nach üblichen Methoden, wie beispielsweise Reduktion, überführt,
und gegebenenfalls die Isomeren trennt, und im Fall der Herstellung der Salze mit einer entsprechenden Base oder Säure umsetzt.

**6.** Arzneimittel enthaltend mindestens ein Phenylglycinamid von heterocyclisch substituierten Phenylessigsäurederivaten.

**7.** Arzneimittel nach Anspruch 6 zur Behandlung von arteriellem Bluthochdruck und Atherosklerose.

**8.** Verfahren zur Herstellung von Arzneimitteln nach Anspruch 6 und 7, dadurch gekennzeichnet, daß man den Wirkstoff gegebenenfalls mit Hilfe von üblichen Hilfs- und Trägerstoffen in eine geeignete Applikationsform überführt.

**9.** Verwendung von Phenylglycinamiden von heterocyclisch substituierten Phenylessigsäurederivaten zur Herstellung von Arzneimitteln.

**10.** Verwendung nach Anspruch 9 zur Herstellung von antiatherosklerotischen Arzneimitteln.

Europäisches Patentamt

# EUROPÄISCHER RECHERCHENBERICHT

Nummer der Anmeldung
EP 94 10 3881

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| A | EP-A-0 513 533 (BAYER AG)<br>* das ganze Dokument, insbesondere Seite 38, Beispiel V, Seite 53, Beispiele LXIV und LXV, Seiten 57-58, Beispiel LXXIV, Seite 62, Beispiel XCVI, Seite 63, Beispiel C, Seiten 85-89, 92-96, 100,102 *<br>--- | 1-10 | C07D233/68<br>C07D233/60<br>C07D233/90<br>C07D401/12<br>A61K31/415 |
| P,X | EP-A-0 560 163 (BAYER AG) 15. September 1993<br>* das ganze Dokument, insbesondere Seite 21, Beispiele 7, 8, Seite 22, Beispiele 9-11, Seite 24, Beispiele 17-19 und Seite 28, Beispiele 38, 39 *<br>--- | 1-10 | |
| A | CHEMICAL ABSTRACTS, vol. 81, no. 25, 23. Dezember 1974, Columbus, Ohio, US; abstract no. 169829k,<br>H. WISSMANN ET AL. 'Chemistry and mechanism of new angiotensin II antagonists. 8-C-Phenylglycine analogs of 5-isoleucine-angiotensin II.'<br>Seite 577 ;<br>* Zusammenfassung *<br>& HOPPE-SEYLER'S Z. PHYSIOL. CHEM. Bd. 355, Nr. 9 , 1974<br>1083 '1096'<br>----- | 1-10 | RECHERCHIERTE SACHGEBIETE (Int.Cl.5)<br>C07D |

Der vorliegende Recherchenbericht wurde für alle Patentansprüche erstellt

| Recherchenort | Abschlußdatum der Recherche | Prüfer |
|---|---|---|
| DEN HAAG | 28. Juni 1994 | Allard, M |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument